# EUROPEAN PATENT SPECIFICATION

(11) **EP 1 815 826 B1**
(45) Date of publication and mention of the grant of the patent: **14.12.2011**
(21) Application number: 07250218.0
(22) Date of filing: 19.01.2007
(51) Int. Cl.: A61F 2/84

(54) **Flexible support member with high pushability**
Leicht drückbares flexibles Trägerteil
Élément de support flexible avec forte poussée

(30) Priority: 24.01.2006 US 338300
(43) Date of publication of application: 08.08.2007
(73) Proprietor: Cordis Corporation, Miami Lakes, Florida 33014 (US)
(72) Inventor: Fernandez, Jose, Coral Springs, Florida 33067 (US); Harries, Heather Ann Bartholf, Penmbroke Pines, FL 33029 (US)
(74) Representative: Belcher, Simon James

(56) References cited:
- EP-A- 1 181 906
- WO-A-01/54761
- US-A1- 2004 230 286

## Description

The present invention is directed to the field of medical devices, and in particular, a system (and components thereof) for delivering and deploying medical implants, such as stents for use in treating disorders such as vascular disease.

A medical implant, such as a stent, is commonly used as a tubular structure left inside the lumen of a duct or vessel to relieve an obstruction. Stents are inserted into the lumen in a non-expanded form and are then expanded *in situ* by self-expanding action or by other means, such as by applying a force extending radially outward to the interior stent walls. Stents may be expanded during or just after balloon angioplasty.

A stent-type medical implant is ordinarily delivered and deployed via a delivery system. Such delivery systems may have an outer sheath forming an elongated tubular member having distal and proximal ends and an inside and outside diameter, and may also include an inner shaft located coaxially within the outer sheath. The inner shaft has a distal end, a proximal end and a longitudinal axis extending therebetween. In many instances, at least a portion of the inner shaft can be made from a flexible coiled member, which permits the delivery device to snake through vessels that have relatively narrow passages and often have curves or bends.

A medical implant is located within the outer sheath, disposed at the distal end of the device, and the inner shaft is disposed coaxially within a lumen of the implant. As the device is moved through the vessels to the deployment site, the flexible coiled member should exhibit sufficient axial stiffness so that the medical implant can be advanced, that is, pushed, to the deployment site.

Thus, the coiled member of the delivery device should possess a degree of flexibility to allow the delivery device to pass through curves and bends in the vessels of a patient's body. However, care must be taken to insure that flexibility is not excessive, for if flexibility is too great, the structural integrity of the coiled member (and hence, the delivery device) could be adversely affected. For example, if flexibility of the coiled member is too great, the device may not be able to move the stent through the vessel. Furthermore, if the flexibility of the coiled member is too great, the coiled member could collapse on itself, in which instance the coiled member might develop kinks that could imperil the medical procedure, if not the patient's health.

EP-A-1181906 discusses a delivery apparatus for a self-expanding stent. The apparatus has an outer sheath and an inner shaft located coaxially within the outer sheath. At least a portion of the inner shaft is made from a flexible coiled member.

In view of the foregoing, it would be desirable to provide a delivery device provided with a shaft having a coiled member portion constructed to provide both a desired degree of flexibility enabling device navigation through tortuous vessel paths, yet exhibiting the axial stability necessary to allow the medical implant to be pushed through the vessel to the deployment site, and sufficient to maintain the necessary rigidity during implant deployment within the vessel and retraction of the device after deployment.

It is an object of the invention to provide a delivery device having a sufficient degree of flexibility, yet possessing sufficient rigidity to permit the device to move, with relative ease, through a body vessel to the deployment site.

According to the present invention, there is provided a pushing member as defined in appended claim 1. The present invention is directed to a pushing member of a delivery device for a medical implant, such as a stent, which delivery device is provided with an inner shaft portion comprised of the pushing member comprising first and second tubes in a coaxial arrangement. In other words, the second, inner tube is positioned inside the first, outer tube. The first and second tubes are cut over at least a segment of the circumference of each respective tube. The cuts in the first and second tubes terminate before the edges of each respective tube, in order to maintain structural integrity of the tubular arrangement. More specifically, the first and second tubes are cut in a helical pattern. The helical pattern is cut at a preselected pitch, that is, an angle, which angle is determined relative to a line drawn perpendicular to the longitudinal axis of the medical device. In a more specific aspect of the present invention, the orientation of the pitch of the cut in the first tube is opposite to the orientation of the pitch of the cut in the second tube (or vice versa).

In accordance with the present invention, a delivery device for a medical implant having at least a portion of the inner shaft comprised of first and second tubes in a coaxial arrangement, as described herein, should exhibit an acceptable degree of stiffness that allows the device to be moved through the vessel by the delivery system, while exhibiting flexibility to allow the device to bend as needed to pass through non linear paths in the patient's body, such as blood vessels having curved or bent portions.

In yet another embodiment, a pushing member for a delivery device for a medical implant may be provided. The pushing member may be a shaft comprised of first and second tubes in a coaxial arrangement can be constructed of first and second metal wires or the like which are wound in a helical pattern at a preselected pitch to create a tubular structure. As above, the first tube is provided with a pitch that runs in a direction opposite to the direction of the pitch of the second tube. The ends of the tube can be welded at the ends to provide the necessary structural integrity. The assembly can be constructed by inserting the second, inner tube within the first, outer tube.

In another aspect, the present invention is directed to a medical device for delivering a medical implant to a site in a patient's body comprising an outer sheath, and a pushing member as defined above.

Furthermore, in a specific aspect of the invention, the orientation of the pitch of the opening in the first tube runs opposite to the orientation of the pitch of the opening in the second tube.

Embodiments of the invention will now be described by way of example with reference to the accompanying drawings, in which
FIG. 1 is an end view of a shaft of a medical device embodying the invention;
FIG. 2 is a cross sectional view of a shaft of a medical device embodying the invention;
FIG. 3 is a perspective view of a medical device that can employ the invention;
FIG. 4 is a perspective view of a the distal end of a shaft of a medical device that can employ the invention.

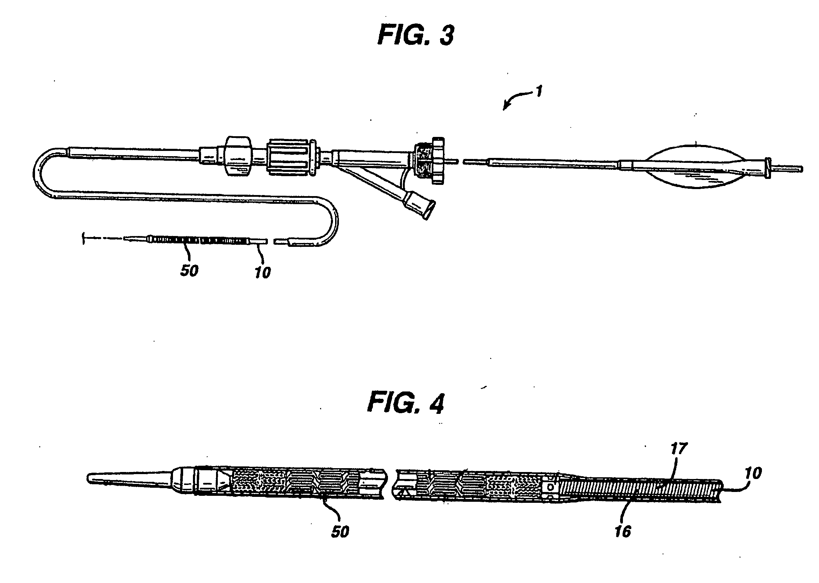

Reference is made to US-6,773,446, which shares the assignee of the present application. Referring now to the figures wherein like numerals indicate the same element throughout the views, there is shown in FIGS. 3 and 4 a medical device delivery device 1, which specifically is a delivery device for self-expanding stents. Device 1 comprises inner and outer coaxial tubes. The inner tube is called the shaft 10 and the outer tube is called the sheath 40. A self-expanding stent 50 is located within the outer sheath, wherein the stent makes frictional contact with the outer sheath and the shaft is disposed coaxially within a lumen of the stent. Shaft 10 has a body portion 16, wherein at least a portion of 16 is made from a flexible coiled member 17.

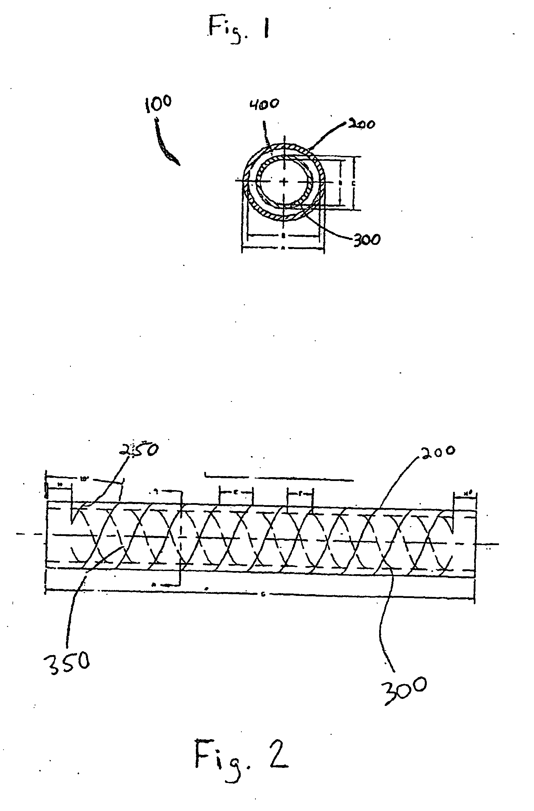

The coaxial tube-in a tube arrangement described herein can be employed as the coiled member portion of the shaft of a medical implant delivery device, which by way of example, can deliver a stent to a site in a vessel in a patient's body in need of treatment. FIGS. 1 and 2 depict the coiled member 100. The coiled member as shown, is cylindrical in its appearance. The stent is constructed of a first and second tubes 200 and 300, respectively, which tubes are characterized by walls that define hollow interiors arranged coaxially, with second tube 300 positioned inside of the first tube 200. Outer tube 200 has a wall thickness defined by outer diameter A and inner diameter B. The inner tube 300 has a wall thickness defined by outer diameter C and inner diameter D. The inner diameter B of the outer first tube 200 is greater than the outer diameter C of second, inner tube 300, so that inner tube 300 can be accommodated within outer tube 200. As shown in Fig. 1, a space or gap 400 is provided between the inner diameter B of the first tube 200 and outer diameter C of the second tube 300. However, the inner tube 300 can be snugly retained in the first outer tube 200 by providing substantially no space, or very little space, between the adjacent walls of the tubes.

As shown in Fig. 2, the outer tube 200 is provided with a cut 250, provided through the thickness of the wall of outer tube 200, which extends around the circumference of the tube and longitudinally along the length of the tube. Tube 300 is also provided with a cut 350 provided through the thickness of the wall of inner tube 300, which cut extends both around the circumference of the tube and longitudinally along the length of the tube. The cuts 250 and 350 in tubes 200 and 300, respectively, have a terminus at location H, which is spaced a distance from a first end of the respective tube. The cuts extend from terminus H both circumferentially and longitudinally, at a preselected pitch, to a location spaced a distance from a second tube end of the respective tube. Preferably, the distance from tube ends to location H and the tube ends to location H' is relatively short when considered in relation to overall tube length. In other words, the cuts should preferably extend over a substantial portion of the lengths of the respective tubes 200 and 300, while leaving the tubes uncut and intact in the region between first tube ends and termini H and second tube ends and termini H'. It should be noted that the distances between the tube ends and the termini need not be equal, and the distance between terminus H and first tube end for tube 200 need not equal the distance between terminus H and first tube end for tube 300. The same arrangement could apply for the distance between H' and the ends of tubes 200 and 300.

As indicated, cuts 250 and 350 in tubes 200 and 300 extend both circumferentially and longitudinally from termini H to H' at a preselected pitch, as determined in relation to an angle perpendicular to the longitudinal axis of the tubes. The pitch of the cuts is preferably about 2° to about 60°, more preferably about 5° to about 45°, and most preferably about 10° to about 25°. In a preferred arrangement the pitch of the cut on first tube 200 is about 10°, and the pitch of the cut on the second tube 300 is also about 10°. In a preferred arrangement, the orientation of the pitch of the cut in the first tube runs opposite to the orientation of the pitch of the cut in the second tube. This arrangement can be seen in Fig. 2.

The selection of the pitch bears upon the longitudinal distances between adjacent cuts, as shown in Fig. 2 as distance E for tube 200 and distance F for tube 300. In other words, the pitch angle can be selected in order to adjust the distance E or F between the cuts, as measured in the direction parallel to the longitudinal axis of the tube. The pitch, and thus, the distance between the cuts, can be modulated to affect the bendability (flexibility) and stiffness (rigidity) of the tube. A pitch that results in the shortening of the distances E and F should result in a tube that is more flexible than one where the distances E and F are spaced further apart. It should be recognized that by varying the pitch with respect to distances along the longitudinal axis of the tube, each tube can be provided with varying regions of stiffness and bendability. Where the tube has a pitch that provides shorter distances between the cuts, the tube will exhibit higher flexibility. In regions where the cuts are spaced apart, the tube will exhibit greater stiffness.

The coiled member of the present invention can be manufactured out of two hypotubes of varying diameters, such that the inner diameter of the first hypotube is sufficiently large as to receive the second hypotube in open space of the first hypotube. The hypotubes can be constructed of any material suitable for implantation in the body of an animal, preferably a mammal, more preferably a human, yet providing a degree of strength sufficient to retain a vessel in an open position. Materials used to construct implantable medical devices, such as stainless steel and cobalt-chromium alloys may be used.

The first and second hypotubes can be cut to the desired length of the medical devices, by conventional means, such as laser etching, electrical discharge machining (EDM), photochemical etching, etc. These techniques can also be employed to make the cuts 250 and 350 in the tubes 200 and 300. After the tube have been cut to size, and cuts 250 and 350 have been made in the tube, tube 300 is inserted within the tube 200.

Alternatively, the tubes can be fabricated from a flat wire. The flat wire can be spiraled around a solid core or the like in desired configuration in order to provide a tube having openings spaced at a preselected distance, in order to provide the desired degrees of bendability and stiffness. The tubes can then be cut to the desired lengths, the ends welded to provide structural integrity and assembled into the final stent product as described above.

It should be understood by a person of ordinary skill that the diameters of the respective tubes, the tube lengths, the pitch of the cuts (or winds) and length of the cuts (and conversely, the uncut lengths at the ends of the tubes) can be varied in order to provide the best structure for a given medical procedure.

With the medical device structure described herein, it is believed that the forces applied by the coiled member of the delivery system to the medical implant are maximized, ensuring the relative ease of movement of the implant-carrying device to the site of implant deployment. At the same time, the arrangement of cuts in the tube-in-a-tube coiled member, with each tube provided with helical cuts or openings extending at pitches opposite to each other, provide the coiled member with flexibility sufficient to allow the coiled member to move though non-linear portions of blood vessels or other body vessels, in which paths with a relatively rigid coiled member might develop a kink. Thus, the present arrangement combines pushability with flexibility. Further, by orienting the pitches in directions opposite to each other, the tendency of the pushing member to collapse or unravel is believed to be overcome.

## Claims

1. A pushing member comprised in an inner shaft coaxial with the outer sheath of a medical device for delivering a medical implant to a site in a patient's body, the pushing member comprising:
a first hollow body (200) of having a periphery, a first end, a second end, and a preselected length; and **characterised in that** the pushing member further comprises:
a second hollow body (300) having a periphery a first end, a second end, and a preselected length;
the second body (300) retained within the interior of the first body (200);
wherein the first and second bodies are each provided with at least one helical opening (250, 350) in each respective periphery, said opening (250, 350) extending around the body and longitudinally for a portion of the length of the respective bodies from a point (H) spaced a first distance from the first end of the respective hollow body to a point (H') spaced a second distance from the second end of the respective hollow body.

2. The pushing member of claim 1 wherein the first and second hollow bodies are cylindrical.

3. The pushing member of claim 2 wherein the openings (250, 350) in the first and second hollow bodies extend at a constant pitch.

4. The pushing member of claim 2 wherein the openings (250, 350) in the first and second hollow bodies extend at pitches that vary with respect to the longitudinal position on the bodies.

5. The pushing member of claim 2 wherein the opening (250) in the first hollow body (200) extends at a pitch of an orientation opposite to the orientation of the pitch of the opening (350) in the second hollow body (300).

6. The pushing member of claim 2 wherein the openings in the first and second hollow bodies extend at a pitch having an angle, measured from a line perpendicular to the axis of the device, that is between about 2° and about 60°.

7. The pushing member of claim 2 wherein the openings in the first and second hollow bodies extend at a pitch having an angle, measured from a line perpendicular to the axis of the device, that is between about 5° and about 45°.

8. The pushing member of claim 2 wherein the openings in the first and second hollow bodies extend at a pitch having an angle, measured from a line perpendicular to the axis of the device, that is between about 10° and about 25°.

9. The pushing member of claim 4 wherein the openings in the first and second hollow bodies extend at a pitch having an angle, measured from a line perpendicular to the axis of the device, that is 10°.

10. A medical device for delivering a medical implant to a site in a patient's body comprising an outer sheath, and a pushing member according to any one of the preceding claims.

## Patentansprüche

1. Schubelement, welches in einem inneren Schaft umfasst ist, welcher koaxial zu der äußeren Hülle einer medizinischen Vorrichtung zum Zuführen eines medizinischen Implantats an einen Ort in dem Körper eines Patienten ist, wobei das Schubelement Folgendes umfasst:
einen ersten Hohlkörper (200), welcher einen Rand, ein erstes Ende, ein zweites Ende und eine vorausgewählte Länge aufweist; und **dadurch gekennzeichnet, dass** das Schubelement weiter Folgendes umfasst:
einen zweiten Hohlkörper (300) mit einem Rand, einem ersten Ende, einem zweiten Ende und einer vorausgewählten Länge;
wobei der zweite Körper (300) in dem Inneren des ersten Körpers (200) zurückgehalten ist;
wobei der erste und der zweite Körper jeweils mit zumindest einer spiralförmiger öffnung (250, 350) in ihrem jeweiligen Rand versehen sind, wobei sich die Öffnung (250, 350) um den Körper und longitudinal über einen Abschnitt der Länge der jeweiligen Körper von einem Punkt (H), welcher einen ersten Abstand von dem ersten Ende des jeweiligen Hohlkörpers enfernt ist, zu einem Punkt (H'), welcher einen zweiten Abstand von dem zweiten Ende des jeweiligen Hohlkörpers entfernt ist, erstreckt.

2. Schubelement nach Anspruch 1, wobei der erste und der zweite Hohlkörper zylindrisch sind.

3. Schubelement nach Anspruch 2, wobei sich die Öffnungen (250, 350) in dem ersten und in dem zweiten Hohlkörper mit einer konstanten Neigung erstrecken.

4. Schubelement nach Anspruch 2, wobei sich die Öffnungen (250, 350) in dem ersten und in dem zweiten Hohlkörper mit Neigungen erstrecken, welche bezüglich der longitudinalen Position auf den Körpern variieren.

5. Schubelement nach Anspruch 2, wobei sich die Öffnung (250) in dem ersten Hohlkörper (200) mit einer Neigung einer der Orientierung der Neigung der Öffnung (350) in dem zweiten Hohlkörper (300) entgegengesetzten Orientierung erstreckt.

6. Schubelement nach Anspruch 2, wobei sich die Öffnungen in dem ersten und in dem zweiten Hohlkörper mit einer Neigung erstrecken, welche einen Winkel aufweist, welcher gemessen von einer Linie senkrecht zu der Vorrichtungsachse zwischen etwa 2° und etwa 60° beträgt.

7. Schubelement nach Anspruch 2, wobei sich die Öffnungen in dem ersten und in dem zweiten Hohlkörper mit einer Neigung erstrecken, welche einen Winkel aufweist, welcher gemessen von einer Linie senkrecht zu der Vorrichtungsachse zwischen etwa 5° und etwa 45° beträgt.

8. Schubelement nach Anspruch 2, wobei sich die Öffnungen in dem ersten und in dem zweiten Hohlkörper mit einer Neigung erstrecken, welche einen Winkel aufweist, welcher gemessen von einer Linie senkrecht zu der Vorrichtungsachse zwischen etwa 10° und etwa 25° beträgt.

9. Schubelement nach Anspruch 4, wobei sich die Öffnungen in dem ersten und in dem zweiten Hohlkörper mit einer Neigung erstrecken, welche einen Winkel aufweist, welcher gemessen von einer Linie senkrecht zu der Vorrichtungsachse 10° beträgt.

10. Medizinische Vorrichtung zum Zuführen eines medizinischen Implantats an einen Ort in dem Körper eines Patienten, welche eine äußere Hülle und ein Schubelement gemäß einem der vorhergehenden Ansprüche umfasst.

## Revendications

1. Élément de poussée compris dans une tige interne coaxiale avec la gaine externe d'un dispositif médical pour poser un implant médical sur un site dans le corps d'un patient, l'élément de poussée comprenant :
un premier corps creux (200) ayant une périphérie, une première extrémité, une deuxième extrémité et une longueur présélectionnée ; et
**caractérisé en ce que** l'élément de poussée comprend en outre :
✔ un deuxième corps creux (300) ayant une périphérie, une première extrémité, une deuxième extrémité et une longueur présélectionnée ;
✔ le deuxième corps (300) étant retenu à l'intérieur du premier corps (200) ;
dans lequel les premier et deuxième corps sont chacun prévus avec au moins une ouverture hélicoïdale (250, 350) dans chaque périphérie respective, ladite ouverture (250, 350) s'étendant autour du corps et longitudinalement sur une partie de la longueur des corps respectifs à partir d'un point (H) espacé selon une première distance de la première extrémité du corps creux respectif jusqu'à un point (H') espacé selon une deuxième distance de la deuxième extrémité du corps creux respectif.

2. Élément de poussée selon la revendication 1, dans lequel les premier et deuxième corps creux sont cylindriques.

3. Élément de poussée selon la revendication 2, dans lequel les ouvertures (250, 350) dans les premier et deuxième corps creux s'étendent à un pas constant.

4. Élément de poussée selon la revendication 2, dans lequel les ouvertures (250, 350) dans les premier et deuxième corps creux s'étendent selon des pas qui varient par rapport à la position longitudinale des corps.

5. Élément de poussée selon la revendication 2, dans lequel l'ouverture (250) dans le premier corps creux (200) s'étend à un pas d'une orientation opposée jusqu'à l'orientation du pas de l'ouverture (350) dans le deuxième corps creux (300).

6. Élément de poussée selon la revendication 2, dans lequel les ouvertures dans les premier et deuxième corps creux s'étendent selon un pas ayant un angle, mesuré à partir d'une ligne perpendiculaire à l'axe du dispositif, qui est compris entre environ 2° et environ 60°.

7. Élément de poussée selon la revendication 2, dans lequel les ouvertures dans les premier et deuxième corps creux s'étendent selon un pas ayant un angle, mesuré à partir d'une ligne perpendiculaire à l'axe du dispositif, qui est compris entre environ 5° et environ 45°.

8. Élément de poussée selon la revendication 2, dans lequel les ouvertures dans les premier et deuxième corps creux s'étendent selon un pas ayant un angle, mesuré à partir d'une ligne perpendiculaire à l'axe du dispositif, qui est compris entre environ 10° et environ 25°.

9. Élément de poussée selon la revendication 4, dans lequel les ouvertures dans les premier et deuxième corps creux s'étendent selon un pas ayant un angle, mesuré à partir d'une ligne perpendiculaire à l'axe du dispositif, qui est de 10°.

10. Dispositif médical pour poser un implant médical sur un site dans le corps d'un patient, comprenant une gaine externe, et un élément de poussée selon l'une quelconque des revendications précédentes.
